# EUROPÄISCHE PATENTSCHRIFT

(11) **EP 0 319 922 B1**
(45) Veröffentlichungstag und Bekanntmachung des Hinweises auf die Patenterteilung: **25.08.1993**
(21) Anmeldenummer: 88120374.9
(22) Anmeldetag: 06.12.1988
(51) Int. Cl.: G01N 21/86, G01N 33/483

(54) **Vorrichtung zur optischen Auswertung eines mindestens ein Testfeld aufweisenden Teststreifens**
Device for the optical evaluation of a test band presenting at least one test field
Dispositif pour la lecture optique d'un ruban test présentant au moins un champ d'épreuve

(30) Priorität: 09.12.1987 DE 8716270 U
(43) Veröffentlichungstag der Anmeldung: 14.06.1989
(73) Patentinhaber: LRE RELAIS + ELEKTRONIK GMBH, D-80335 München (DE)
(72) Erfinder: Gassenhuber, Helmut, D-8130 Starnberg (DE)
(74) Vertreter: Schaumburg, Thoenes & Thurn

(56) Entgegenhaltungen:
- EP-A- 0 037 484
- EP-A- 0 154 875
- EP-A- 0 182 647
- EP-A- 0 183 524
- EP-A- 0 253 371
- WO-A-83/00926
- DE-U- 8 716 270

## Beschreibung

Die Erfindung betrifft eine Vorrichtung zur optischen Auswertung eines mindestens ein Testfeld aufweisenden Teststreifens, umfassend ein Gehäuse mit einem Aufnahmeschacht für den Teststreifen, eine Meßoptik mit einem Sender zum Beleuchten des Testfeldes und einem Empfänger zum Empfangen des vom Testfeld remittierten Lichtes und eine elektronische Auswerte- und Anzeigevorrichtung. Die Vorrichtung ist insbesondere zum Untersuchen des Cholesteringehaltes von Blut bestimmt.

Eine zuverlässige Messung mit Hilfe der vorstehend genannten Vorrichtung setzt voraus, daß die Meßvorrichtung nicht von vorhergehenden Messungen verunreinigt ist und daß die Teststreifen stets eine definierte Lage relativ zur Meßoptik einnehmen.

Die EP-A- 253 371 zeigt eine Vorrichtung zur optischen Auswertung eines mindestens ein Testfeld aufweisenden Teststreifens, umfassend ein Gehäuse mit einem Aufnahmeschacht für den Teststreifen, eine Meßoptik mit einem Sender zum Beleuchten des Testfeldes und einem Empfänger zum Empfang des von dem Testfeld remittierten Lichtes, eine elektronische Auswerte- und Anzeigevorrichtung, einen in das Gehäuse lösbar einschiebbaren Teststreifenhalter mit einer Aufnahmerinne für den Teststreifen, deren Breite annähernd der Breite des Teststreifens entspricht, eine Stützplatte mit einer Meßöffnung und ein beweglich gelagertes Andruckelement, das mittels eines Schiebers zwischen einer ersten Stellung, in der es das Einführen und Herausnehmen eines Teststreifens erlaubt, und einer zweiten Stellung verstellbar ist, in der es den Teststreifen an die Stützplatte andrückt.

Die Erfindung liegt die Aufgabe zugrunde, eine Vorrichtung der eingangs genannten Art so auszubilden, daß alle Bauteile, die mit dem Streifen in Berührung kommen, bequem gereinigt werden können und daß eine präzise Ausrichtung des Teststreifens relativ zur Meßoptik gewährleistet ist.

Diese Aufgabe wird erfindungsgemäß gelöst durch einen in den Aufnahmeschacht lösbar einschiebbaren Teststreifenhalter mit einer Aufnahmerinne für den Teststreifen, deren Breite auf mindestens einem Teil ihrer Länge der Breite des Teststreifens entspricht, eine in einem Abstand vom Rinnenboden am Teststreifenhalter angeordnete, eine Meßöffnung aufweisende Stützplatte, ein in der Aufnahmerinne unterhalb der Stützplatte beweglich gelagertes Andruckelement, das zwischen einer ersten Stellung, in der es im wesentlichen mit dem Rinnenboden fluchtet, und einer zweiten Stellung verstellbar ist, in der es den Teststreifen an die Stützplatte andrückt, und eine Andruckvorrichtung zum Verstellen des Andruckelementes zwischen seiner ersten und seiner zweiten Stellung.

Bei der erfindungsgemäßen Lösung kommen die mit der zu untersuchenden Flüssigkeit und den Reaktionssubstanzen versehenen Teile des Teststreifens nur mit dem Teststreifenhalter und den an ihm angeordneten Teilen in Berührung. Der Teststreifenhalter kann jedoch als Ganzes aus der Vorrichtung entfernt und damit bequem gereinigt werden, so daß die Messungen nicht durch Verschmutzungen beeinträchtigt werden, die von vorhergehenden Messungen herrühren. Durch das Andrücken des Teststreifens an die Stützplatte wird gleichzeitig eine definierte Lage des Teststreifens relativ zur Meßoptik gewährleistet. Dabei ist es auch möglich, durch Andrücken des Andruckelementes ohne Teststreifen die Meßöffnung in der Stützplatte zu verschließen und damit eine Dunkelkalibrierung der Vorrichtung vorzunehmen. Ferner kann durch Öffnen des Vorrichtungsgehäuses bei eingelegtem Teststreifen die zu untersuchende Flüssigkeit auf ein Aufnahmefeld des Teststreifens aufgebracht werden, ohne daß der Teststreifen noch einmal seine Lage verändern muß. Damit ist es möglich, auch eine Leerwerteichung vorzunehmen, d.h. eine Vergleichsmessung unter identischen Meßbedingungen an dem Teststreifen ohne die aufgetropfte, zu untersuchende Substanz.

Um den Teststreifen nicht nur gegen eine seitliche Verschiebung, sondern auch gegen eine Verschiebung in seiner Längsrichtung zu sichern, ist es zweckmäßig, wenn nahe dem inneren Ende des Aufnahmeschachtes und innerhalb der Vorrichtung ein Anschlag für den Teststreifen sowie eine Klemmvorrichtung zum kraftschlüssigen Festhalten des Teststreifens im eingeschobenen Zustand vorgesehen ist.

Zweckmäßigerweise steigt der Boden der Aufnahmerinne von dem Einschubende derselben zur Stützplatte hin an. Wird der Teststreifen nur in die Aufnahmerinne eingelegt, aber noch nicht an die Stützplatte angedrückt, so besteht in dieser Lage des Teststreifens die Möglichkeit, das Testfeld zu belüften. Dies kann noch dadurch sichergestellt werden, daß in einem den Aufnahmeschacht abdeckenden Gehäusedeckel nahe dem Einschubende des Aufnahmeschachtes ein in Richtung auf den Rinnenboden des Teststreifenhalters vorgespannter Andruckstift verschiebbar gelagert ist, welcher den Teststreifen gegen den Rinnenboden drückt, solange der Teststreifen nicht durch die Andruckvorrichtung gegen die Stützplatte gedrückt wird.

Um auch hinsichtlich der Temperatur gleiche Meßbedingungen für alle Messungen zu schaffen, ist erfindungsgemäß vorgesehen, daß die Stützplatte und das Andruckelement mittels einer regelbaren Heizeinrichtung beheizbar sind. Da das Testfeld zwischen der Stützplatte und dem Andruckelement eingespannt ist, wird seine Temperatur durch die Temperatur dieser beiden Bauteile bestimmt, da der Teststreifen in diesem Bereich praktisch keinen Wärmekontakt mit der Umgebung hat.

Gemäß einer bevorzugten Ausführungsform ist die Stützplatte als Quersteg eines U-förmigen Stützbauteiles ausgebildet, dessen U-Schenkel zum Rinnenboden hinweisen. Das Andruckelement weist ebenfalls einen U-förmigen Querschnitt auf und ist parallel zu dem Stützbauteil zwischen dessen U-Schenkeln angeordnet, wobei die U-Schenkel des Andruckelementes einen Abstand von den U-Schenkeln des Stützbauteiles haben. Schließlich ist ein U-förmiger Heizelemententräger vorgesehen, an dessen U-Quersteg ein Heizelement angeordnet ist und dessen U-Schenkel zwischen den U-Schenkeln des Andruckelementes und des Stützbauteiles eingeschlossen sind. Der Heizelemententräger ist dabei gehäusefest. Insbesondere wenn das Stützbauteil und das Andruckelement aus einem die Wärme gut leitenden Material, beispielsweise Metall bestehen, kann die Heizenergie von dem Heizelement gut auf diese Bauteile übertragen werden. Der Teststreifenhalter ist im übrigen vorzugsweise aus Kunststoff hergestellt, da dieses Material leicht gereinigt werden kann und im übrigen gute thermische Isoliereigenschaften besitzt.

Weitere Merkmale und Vorteile der Erfindung ergeben sich aus den weiteren Unteransprüchen und der folgenden Beschreibung, welche in Verbindung mit den beigefügten Zeichnungen die Erfindung anhand eines Ausführungsbeispieles erläutert. Es zeigen:
- Fig. 1: eine schematische Gesamtansicht eines erfindungsgemäßen Meßgerätes,
- Fig. 2: einen teilweise schematischen Teilschnitt durch das Gerät entlang Linie II-II in Figur 1, wobei ein Teststreifen in das Gerät eingeschoben ist,
- Fig. 3: eine der Figur 2 entsprechende Ansicht mit einem eingeschobenen und an die Meßoptik angedrückten Teststreifen,
- Fig. 4: eine schematische, perspektivische Darstellung des Teststreifenhalters allein und
- Fig. 5: einen Teilschnitt durch das Gerät entlang Linie V-V in Figur 3.

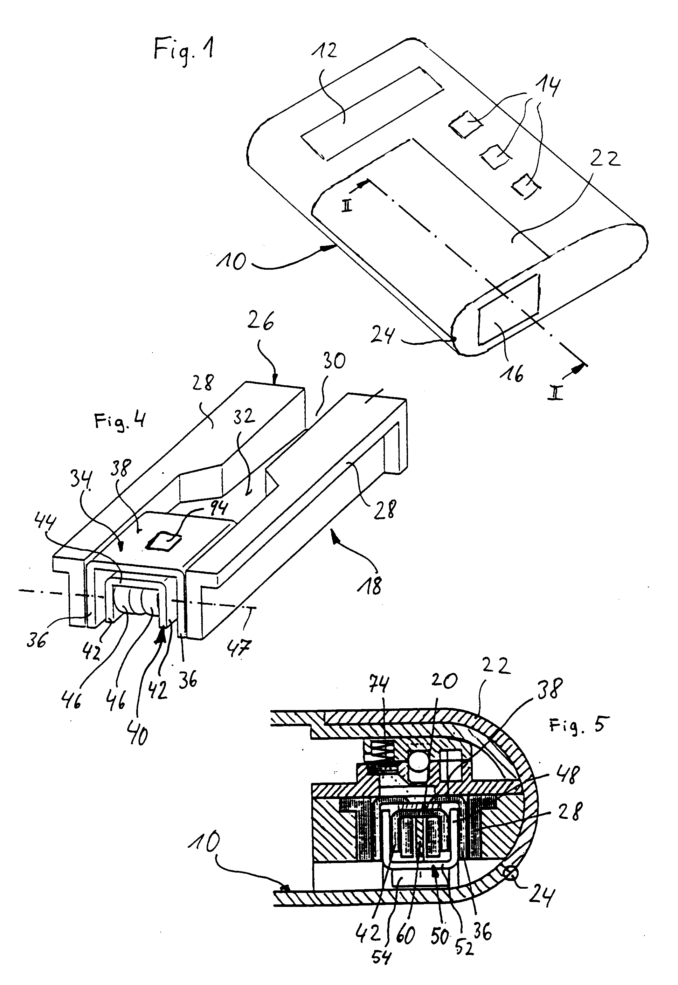

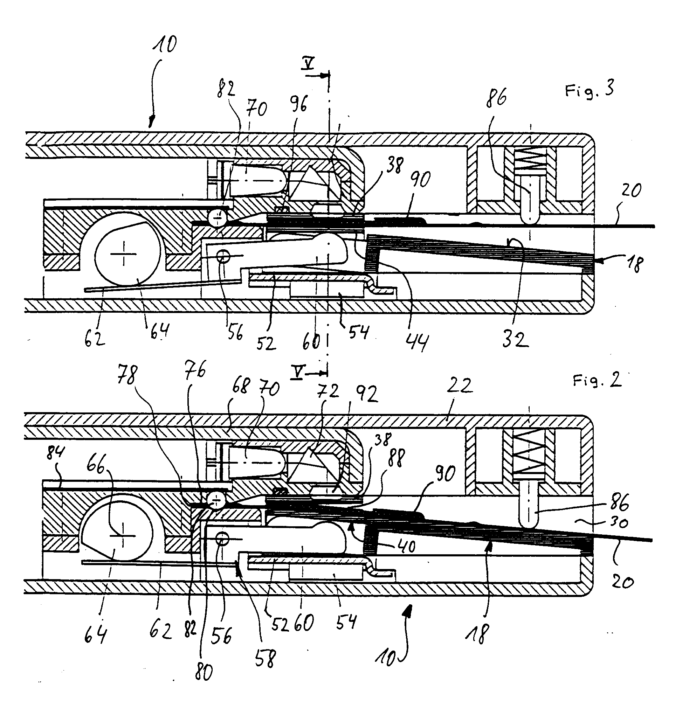

Das in der Figur 1 dargestellte Gerät dient beispielsweise zur Untersuchung des Cholesteringehaltes von Blut durch eine Remissionsmessung mittels einer im Gerät vorgesehenen Meßoptik. Das Gerät umfaßt ein allgemein mit 10 bezeichnetes Gehäuse, an dessen Oberseite ein Anzeigefeld 12 und Bedienungselemente 14 zu erkennen sind. Das Anzeigeelement 12 und die Bedienungselemente 14 sind nur schematisch angedeutet. Sie brauchen in Zahl und Anordnung nicht mit der Anordnung bei einem tatsächlichen Gerät übereinzustimmen. Ihre Anordnung spielt für die Erläuterung der vorliegenden Erfindung keine Rolle. An dem zum Betrachter weisenden Ende des Gehäuses 10 erkennt man einen Aufnahmeschacht 16 zur Aufnahme eines in Figur 4 dargestellten Teststreifenhalters 18, in den wiederum in noch zu beschreibender Weise ein Teststreifen 20 eingeschoben werden kann (Figuren 2 und 3). Oberhalb des Aufnahmeschachtes 16 befindet sich in dem Gehäuse 10 ein Klappdeckel 22, der um eine Achse 24 verschwenkt werden kann, um so ein Auftropffeld auf dem im Teststreifenhalter 18 liegenden Teststreifen 20 freizugeben, wie dies anhand der Figuren 2 und 3 noch näher erläutert wird.

Der in der Figur 4 dargestellte Teststreifenhalter 18 umeinstückiges Kunststoffteil 26 mit zwei seitlichen Profilschenkeln 28, die zwischen sich eine Aufnahmerinne 30 zur Aufnahme eines Teststreifens 20 einschließen, wobei der Rinnenboden 32 gemäß den Darstellungen in den Figuren 2 und 3 vom Einschubende des Teststreifenhalters 18 zu seinem zum Gehäuseinneren hinweisenden anderen Längsende hin ansteigt. Der Rinnenboden 32 bildet so eine Rampe, auf der der Teststreifen 20 in das Gerät eingeschoben werden kann, wobei die Breite des unmittelbar an das Einschubende angrenzenden Abschnittes der Aufnahmerinne 30 im wesentlichen der Breite des Teststreifens 20 entspricht, so daß dieser seitlich geführt ist.

An dem zum Geräteinneren hinweisenden Längsende des Teststreifenhalters 18 ist zwischen den freien Enden der Profilschienen 28 ein U-förmiges Stützbauteil 34 fest angeordnet, das mit seinen U-Schenkeln 36 an den benachbarten Profilschienen 28 anliegt und mit seinem eine Stützplatte bildenden U-Quersteg 38 mit den oberen Rändern der Profilschienen 28 fluchtet. Das Stützbauteil 34 ist fest mit dem Kunststoffteil 26 verbunden.

Innerhalb des U-förmigen Stützbauteils 34 ist ein U-förmiges Andruckelement 40 angeordnet, das mit seinen U-Schenkeln 42 und seinem U-Quersteg 44 jeweils im wesentlichen parallel zu den entsprechenden Teilen des Stützbauteiles 34 gerichtet ist, von diesen jedoch jeweils einen Abstand aufweist. Das Andruckelement 40 ist an Fortsätzen 46 des Kunststoffteils 26 um eine in Figur 4 eingezeichnete Achse 47 schwenkbar gelagert, so daß es in einer ersten für die Aufnahme eines Teststreifens 20 geeigneten Stellung mit dem rampenförmigen Rinnenboden 32 fluchtet (Figur 2) und in einer der Andruckstellung des Teststreifens 20 an die Stützplatte 38 entsprechenden zweiten Stellung parallel zu der Stützplatte 38 liegt (Figur 3). Auch das Andruckelement 40 ist vorzugsweise aus Metall hergestellt.

Ist der Teststreifenhalter 18 in den Aufnahmeschacht 16 des Gehäuses 10 eingeschoben (Figuren 2, 3 und 5), so greifen zwischen die U-Schenkel 36 und 42 des Stützbauteiles 34 und des Andruckelementes 40 jeweils U-Schenkel 48 eines U-förmigen Heizelemententrägers 50, der unterhalb des Aufnahmeschachtes 16 in dem Gehäuse 10 angeordnet ist und auf der dem Teststreifenhalter 18 fernen Außenseite seines U-Quersteges 52 ein Heizelement 54 trägt. Durch Wärmeleitung und Wärmestrahlung überträgt der Heizelemententräger Wärme auf das Andruckelement 40 und das Stützbauteil 34.

Nahe dem inneren Ende des Aufnahmeschachtes 16 ist um eine Achse 56 ein zweiarmiger Andruckhebel 58 schwenkbar gelagert. Er greift mit seinem einen Hebelarm 60 zwischen die Fortsätze 46 unter den U-Quersteg 44 des Andruckelementes 40. Sein entgegengesetzter Hebelarm ist von einer Blattfeder 62 gebildet, die am Umfang einer Nockenscheibe 64 anliegt, die um eine Achse 66 drehbar in dem Gehäuse 10 gelagert ist.

Oberhalb des Aufnahmeschachtes 16 befindet sich in einem Innengehäuse 58 eine Meßoptik für die Durchführung der Remissionsmessung mit einem Sender 70, einem Umlenkprisma 72 und einem Empfänger 74 (Figur 5).

Nahe dem inneren Ende des Aufnahmeschachtes befindet sich ferner zwischen zwei gehäusefesten Flächen in Verlängerung der Aufnahmerinne 30 ein Schlitz 76, dessen Endfläche 78 einen Anschlag für das in Einschubrichtung weisende Ende des Teststreifens 20 bildet, wenn dieser in den Aufnahmeschacht bzw. die Aufnahmerinne des Teststreifenhalters 18 eingeschoben wird. In der eingeschobenen Stellung (Figuren 2 und 3) wird der Teststreifen durch eine Klemmvorrichtung kraftschlüssig gehalten, die eine Kerbe 80 in der einen den Spalt 76 begrenzenden Fläche und eine der Kerbe 80 zugeordnete Rastkugel 82 in der anderen den Spalt 76 begrenzenden Fläche umfaßt, wobei diese Rastkugel 82 beispielsweise durch eine Blattfeder 84 in Richtung auf die Kerbe 80 vorgespannt ist.

Der gemäß Figur 2 bis zum Anschlag an der Anschlagfläche 78 eingeschobene Teststreifen 20 liegt zunächst flach auf dem Rinnenboden 32 auf, wobei er durch einen in dem Gehäusedeckel 22 federnd gelagerten Andruckstift 86 gegen den Rinnenboden 32 gehalten wird. Die Abmessungen sind so gewählt, daß ein Testfeld 88 des Teststreifens 20 genau zwischen der Stützplatte 38 und dem U-Quersteg 44 des Andruckelementes 40 liegt, wenn der Teststreifen 20 vollständig eingeschoben ist. Das sich an das Testfeld 88 anschließende Auftropffeld 90 liegt dabei frei. Zur Durchführung einer Messung wird nun die Nockenscheibe 64 aus der in der Figur 2 dargestellten Stellung in die in der Figur 3 dargestellten Stellung verdreht, wodurch der Andruckhebel 38 im Gegenuhrzeigersinn verschwenkt wird und dabei das Andruckelement 40 mit seinem U-Quersteg 44 zusammen mit dem Teststreifen 20 flach gegen die Unterseite der Stützplatte 38 drückt. Das Testbild 88 befindet sich damit in einer wohl definierten Lage relativ zur Meßoptik 70, 72 und 74. Der Strahlengang von dem Sender 70 verläuft durch das Prisma 72 und eine Öffnung 92 in dem die Optik aufnehmenden Gehäuse sowie durch ein Fenster 94 in der Stützplatte 38 zum Testfeld 38, von wo Strahlen zum Empfänger 74 hin remittiert werden. Wird kein Teststreifen 20 eingelegt, und das Fenster 94 durch das Andrücken des Andruckelementes 40 verschlossen, so kann eine Dunkelkalibrierung des Gerätes vorgenommen werden. Ferner kann nach dem Andrücken des Teststreifens 20 (Figur 3) zunächst eine Leereichung erfolgen, bevor dann auf das Auftropffeld 90 die zu untersuchende Flüssigkeit aufgetropft werden kann. Das Auftropffeld ist dabei durch das Öffnen des Deckels 22 frei zugänglich. Während der Reaktion, die nach dem Auftropfen der zu untersuchenden Flüssigkeit aber vor der Messung im Testfeld 88 stattfindet, wird dieser Bereich des Teststreifens 20 mittels der Heizvorrichtung 50, 54 auf einer vorbestimmten Temperatur gehalten. Dabei sorgt ein Temperaturfühler 96, der im eingeschobenen Zustand des Teststreifenhalters 18 mit der Stützplatte 38 in Berührung tritt, in Verbindung mit einer nicht dargestellten Regeleinrichtung dafür, daß eine konstante vorgegebene Temperatur eingehalten wird. Soll das Testfeld belüftet werden, so wird der Andruckhebel aus der in der Figur 3 dargestellten Stellung wieder in die in der Figur 2 dargestellte Stellung durch das Drehen der Nockenscheibe 64 verstellt, wobei der Stift 86 den Teststreifen 20 gegen den Rinnenboden 32 drückt und dafür sorgt, daß das Testbild 88 sich von der Stützplatte 38 löst. In diesem Zustand kann der Teststreifen 20 auch aus der Vorrichtung wieder herausgenommen werden.

Die vorstehende Beschreibung zeigt, daß sämtliche Flächen, die mit dem Testfeld 88 und dem Auftropffeld 90 des Teststreifens 20 in Berührung kommen, an den Teststreifenhalter 18 angeordnet sind, der aus dem Gerät 10 herausgezogen und anschließend bequem gereinigt werden kann. Lediglich das über das Testfeld 88 hinausragende vordere Ende des Teststreifens kommt mit Teilen innerhalb des Gerätes in Berührung, wobei jedoch eine Verschmutzung in diesem Bereich die Meßergebnisse nicht beeinflussen kann. Der Teststreifen kann auf einfache Weise in eine stets gleichbleibende definierte Lage für die Messung gebracht werden, wobei gleichzeitig seine Temperatur auf einem vorgegebenen stabilisierten Wert gehalten werden kann. In Verbindung mit der bequemen Möglichkeit einer Dunkelkalibrierung der Meßvorrichtung und einer Leereichung erlaubt die vorstehend beschriebene erfindungsgemäße Anordnung somit die Durchführung exakter und reproduzierbarer Messungen.

## Patentansprüche

1. Vorrichtung zur optischen Auswertung eines mindestens ein Testfeld (88) aufweisenden Teststreifens (20), umfassend ein Gehäuse (10) mit einem Aufnahmeschacht (16) für den Teststreifen (20), eine Meßoptik (70, 72, 74) mit einem Sender (70) zum Beleuchten des Testfeldes (88) und einem Empfänger (74) zum Empfang des vom Testfeld (88) remittierten Lichtes, eine elektronische Auswerte -und Anzeigevorrichtung, einen in den Aufnahmeschacht (16) lösbar einschiebbaren Teststreifenhalter (18) mit einer Aufnahmerinne (30) für den Teststreifen (20), deren Breite auf mindestens einem Teil ihrer Länge der Breite des Teststreifens (20) entspricht, eine in einem Abstand vom Rinnenboden (32) am Teststreifenhalter (18) angeordnete, eine Meßöffnung (94) aufweisende Stützplatte (38), ein in der Aufnahmerinne (30) unterhalb der Stützplatte (38) beweglich gelagertes Andruckelement (40), das zwischen einer ersten Stellung, in der es im wesentlichen mit dem Rinnenboden (32) fluchtet, und einer zweiten Stellung verstellbar ist, in der es den Teststreifen (20) an die Stützplatte (38) andrückt, und eine Andruckvorrichtung (58, 64) zum Verstellen des Andruckelementes (40) zwischen seiner ersten und seiner zweiten Stellung.

2. Vorrichtung nach Anspruch 1, dadurch **gekennzeichnet,** daß nahe dem inneren Ende des Aufnahmeschachtes (16) ein Anschlag (78) für den Teststreifen (20) sowie eine Klemmvorrichtung (80, 82) zum kraftschlüssigen Festhalten des Teststreifens (20) im eingeschobenen Zustand vorgesehen ist.

3. Vorrichtung nach Anspruch 1 oder 2, dadurch **gekennzeichnet,** daß der Boden (32) der Aufnahmerinne (30) von dem Einschubende derselben zur Stützplatte (38) hin ansteigt.

4. Vorrichtung nach einem der Ansprüche 1 bis 3, dadurch **gekennzeichnet,** daß die Stützplatte (38) und das Andruckelement (40) mittels einer regelbaren Heizeinrichtung (50, 54, 96) beheizbar sind.

5. Vorrichtung nach Anspruch 4, dadurch **gekennzeichnet,** daß die Stützplatte (38) als Quersteg eines U-förmigen Stützbauteiles (34) ausgebildet ist, dessen U-Schenkel (36) zum Rinnenboden (32) hinweisen, daß das Andruckelement (40) einen U-förmigen Querschnitt aufweist und parallel zu dem Stützbauteil (34) zwischen dessen U-Schenkeln (36) angeordnet ist, wobei die U-Schenkel (42) des Andruckelementes (40) einen Abstand von den U-Schenkeln (36) des Stützbauteiles (34) haben, und daß ein U-förmiger Heizelemententräger (50) vorgesehen ist, an dessen U-Quersteg (52) ein Heizelement (54) angeordnet ist und dessen U-Schenkel (48) zwischen den U-Schenkeln (42 bzw. 36) des Andruckelementes (40) und des Stützbauteiles (34) eingeschlossen sind.

6. Vorrichtung nach Anspruch 5, dadurch **gekennzeichnet**, daß das Stützbauteil (34) und das Andruckelement (40) aus einem die Wärme gut leitenden Material bestehen.

7. Vorrichtung nach einem der Ansprüche 1 bis 6, dadurch **gekennzeichnet,** daß die Andruckvorrichtung einen zweiarmigen Schwenkhebel (58) umfaßt, der mit seinem einen Hebelende (60) an der dem Teststreifen (20) abgewandten Seite des Andruckelementes (40) anliegt und über eine an seinem anderen Hebelende (62) angreifende Steuerkurve (64) zwischen einer Andruckstellung und einer Freigabestellung verstellbar ist.

8. Vorrichtung nach Anspruch 7, dadurch **gekennzeichnet,** daß einer der Hebelarme (62, 60) als Federschenkel ausgebildet ist.

9. Vorrichtung nach Anspruch 7 oder 8, dadurch **gekennzeichnet,** daß die Steuerkurve (64) durch einen Antriebsmotor verstellbar ist.

10. Vorrichtung nach einem der Ansprüche 1 bis 9, dadurch **gekennzeichnet,** daß in einem den Aufnahmeschacht (16) abdeckenden Gehäusedeckel (22) nahe dem Einschubende des Aufnahmeschachtes (16) ein in Richtung auf den Rinnenboden (32) des Teststreifenhalters (18) vorgespannter Andruckstift (86) gelagert ist.

11. Vorrichtung nach einem der Ansprüche 1 bis 10, dadurch **gekennzeichnet,** daß der Teststreifenhalter aus Kunststoff besteht.

## Claims

1. A device for optically evaluating a test strip (20) comprising at least one test area (88), the device comprising a casing (10) having a shaft (16) for receiving the test strip (20), a measuring optical system (70, 72, 74) comprising a transmitter (70) for illuminating the test area (88) and a receiver (74) for receiving light sent back from the test area (88), an electronic evaluating and display device, a test-strip holder (18) loosely insertable into the shaft (16) and comprising a channel (30) for receiving the test strip (20), the width of the channel along at least part of its length corresponding to the width of the test strip (20), a supporting plate (38) formed with a measuring opening (94) and disposed on the test-strip holder (18) at a distance from the channel bottom (32), a pressure element (40) mounted for movement in the channel (30) underneath the supporting plate (38) and adjustable between a first position in which it is substantially in line with the channel bottom (32) and a second position in which it presses the test strip (20) against the supporting plate (38), and a pressure device (58, 64) for adjusting the pressure element (40) between its first and its second position.

2. A device according to claim 1, characterised in that an abutment (78) for the test strip (20) and a clamping device (80, 82) for non-positively holding the test strip (20) when inserted are provided near the inner end of the shaft (16).

3. A device according to claim 1 or 2, characterised in that the bottom (32) of the channel (30) rises from the insertion end towards the supporting plate (38).

4. A device according to any of claims 1 to 3, characterised in that the supporting plate (38) and the pressure element (40) are heatable by an adjustable heating device (50, 54, 96).

5. A device according to claim 4, characterised in that the supporting plate (38) is the transverse web of a U-shaped supporting component (34), the arms (36) of the U pointing towards the channel bottom (32), the pressure element (40) has a U-shaped cross-section and is disposed parallel to the supporting component (34) between the arms (36) of the U, the arms (42) of the U-shaped pressure element (40) being at a distance from the arms (36) of the supporting component (34), and a U-shaped heating-element holder (50) is provided, a heating element (54) being disposed on the cross-member (52) of the U and the arms (48) of the U being enclosed between the arms (42, 36) of the pressure element (40) and the supporting component (34).

6. A device according to claim 5, characterised in that the supporting component (34) and the pressure element (40) are made of a material which is a good conductor of heat.

7. A device according to any of claims 1 to 6, characterised in that the pressure device comprises a two-armed rocking lever (58), one end (60) of the lever abutting the side of the pressure element (40) remote from the test strip (20) and a cam (64) engaging the other end (62) of the lever so as to adjust it between a pressure position and a release position.

8. A device according to claim 7, characterised in that one arm (62, 60) of the lever is a spring arm.

9. A device according to claim 7 or 8, characterised in that the cam (64) is adjustable by a drive motor.

10. A device according to any of claims 1 to 9, characterised in that a pressure pin (86) prestressed towards the channel bottom (32) of the test-strip holder (18) is disposed near the insertion end of the shaft (16) in a casing cover (22) which covers the shaft (16).

11. A device according to any of claims 1 to 10, characterised in that the test-strip holder is made of plastics.

## Revendications

1. Dispositif d'analyse optique d'une bande de test (20) incluant au moins une zone de test (88), comprenant un boîtier (10) comportant un canal (16) de réception de la bande de test (20), une optique de mesure (70, 72, 74) comprenant un émetteur (70) d'illumination de la zone de test (88) et un récepteur (74) destiné à recevoir la lumière réfléchie par la zone de test (88), un dispositif électronique d'analyse et d'affichage, un support (18) de la bande de test pouvant être introduit de manière amovible dans le canal de réception (16) et comprenant un couloir (30) de logement de la bande de test (20), dont la largeur correspond sur au moins une partie de sa longueur à la largeur de la bande de test (20), une plaque d'appui (38) disposée à distance du fond (32) du couloir sur le support (18) de la bande de test et comportant un trou de mesure (94), un élément de pression (40) monté mobile dans le couloir de logement (30) sous la plaque d'appui (38) et déplaçable entre une première position à laquelle il est sensiblement à l'alignement du fond (32) du couloir et une seconde position à laquelle il serre la bande de test (20) contre la plaque d'appui (38), ainsi qu'un dispositif de pression (58, 64) destiné à déplacer l'élément de pression (40) entre sa première et sa seconde position.

2. Dispositif selon la revendication 1, caractérisé en ce qu'une butée (78) de la bande de test (20), ainsi qu'un dispositif de serrage (80, 82) destiné à retenir sous la contrainte d'une force la bande de test (20) à l'état dans lequel elle a été introduite, sont prévus à proximité de l'extrémité intérieure du canal de réception (16).

3. Dispositif selon la revendication 1 ou 2, caractérisé en ce que le fond (32) du couloir de logement (30) est ascendant de son extrémité d'introduction vers la plaque d'appui (38).

4. Dispositif selon l'une des revendications 1 à 3, caractérisé en ce que la plaque d'appui (38) et l'élément de pression (40) sont chauffables au moyen d'un dispositif réglable de chauffage (50, 54, 96).

5. Dispositif selon la revendication 4, caractérisé en ce que la plaque d'appui (38) est constituée de l'âme d'un composant d'appui en étrier (34) dont les ailes (36) sont orientées vers le fond (32) du couloir, en ce que l'élément de pression (40) a une section en étrier et est disposé parallèlement au composant d'appui (34) entre ses ailes (36), les ailes (42) de l'élément de pression (40) étant à distance des ailes (36) du composant d'appui (34) et en ce qu'un support en étrier (50) d'élément chauffant est prévu, un élément chauffant (54) étant disposé sur son âme (52) et les ailes (48) du support étant enveloppées par les ailes (42 et respectivement 36) de l'élément de pression (40) et du composant d'appui (34).

6. Dispositif selon la revendication 5, caractérisé en ce que le composant d'appui (34) et l'élément de pression (40) sont en un matériau bon conducteur de la chaleur.

7. Dispositif selon l'une des revendications 1 à 6, caractérisé en ce que le dispositif de pression comprend un levier oscillant (58) à deux bras, dont une extrémité (60) est appliquée contre le côté de l'élément de pression (40) qui est tourné à l'opposé de la bande de test (20) et qui est déplaçable par une came (64) attaquant son autre extrémité (62) entre une position de pression et une position de libération.

8. Dispositif selon la revendication 7, caractérisé en ce que l'un des bras (62, 60) du levier est conformé en branche de ressort.

9. Dispositif selon la revendication 7 ou 8, caractérisé en ce que la came (64) est déplaçable par un moteur de commande.

10. Dispositif selon l'une des revendications 1 à 9, caractérisé en ce qu'une tige de pression (86) mise sous précontrainte vers le fond (32) du couloir du support (18) de la bande de test est montée à proximité de l'extrémité d'entrée du canal de réception (16) dans un couvercle (22) du boîtier qui recouvre le canal de réception (16).

11. Dispositif selon l'une des revendications 1 à 10, caractérisé en ce que le support de la bande de test est en matière plastique.
